Europäisches Patentamt

European Patent Office

Office européen des brevets

(11) Veröffentlichungsnummer: **0 190 762 B1**

(12) **EUROPÄISCHE PATENTSCHRIFT**

(45) Veröffentlichungstag der Patentschrift: **15.04.92**

(21) Anmeldenummer: **86101559.2**

(22) Anmeldetag: **06.02.86**

Die Akte enthält technische Angaben, die nach dem Eingang der Anmeldung eingereicht wurden und die nicht in dieser Patentschrift enthalten sind.

(51) Int. Cl.5: **A61K 7/16**

(54) **Zahnpasta.**

(30) Priorität: **07.02.85 DE 3504178**

(43) Veröffentlichungstag der Anmeldung:
**13.08.86 Patentblatt 86/33**

(45) Bekanntmachung des Hinweises auf die Patenterteilung:
**15.04.92 Patentblatt 92/16**

(84) Benannte Vertragsstaaten:
**AT BE DE FR GB IT LU NL SE**

(56) Entgegenhaltungen:
**DE-C- 629 450**
**FR-A- 498 374**
**FR-A- 2 251 309**
**GB-A- 1 476 063**
**US-A- 4 486 404**

(73) Patentinhaber: **Blendax GmbH**
**Rheinallee 88**
**W-6500 Mainz 1(DE)**

(72) Erfinder: **Frosch, Franz, Dr.**
**Eddersbacher Berg 6**
**W-6204 Taunusstein(DE)**
Erfinder: **Harth, Helmut, Dr.**
**Lilienweg 24**
**W-6500 Mainz 21(DE)**

**Beschreibung**

Die vorliegende Erfindung betrifft eine Zahnpasta mit verbesserten remineralisierenden Eigenschaften, die Calciumcarbonat als Poliermittel enthält, und deren pH-Wert zwischen etwa 7,5 und etwa 8,5 liegt.

Es ist in der Zahnmedizin bereits seit langem bekannt, daß durch Zuführung von Calcium- sowie ggf. Phosphat- und Fluorid-Ionen eine Remineralisierung von demineralisierten Stellen im Kristallgitter des Zahnschmelzes erfolgen kann.

In der Praxis wird von dieser Erkenntnis dadurch Gebrauch gemacht, daß man Zahnpflegemitteln, insbesondere Zahnpasten, Calciumverbindungen zusetzt; die durch Dissoziation in wässrigem Medium freiwerdenden Calciumionen bewirken beim Kontakt mit demineralisierten Zahnschmelzoberflächen eine Remineralisierung durch ihren Einbau in die freien Stellen des Kristallgitters des Schmelzes.

Zur Erreichung dieser Wirkung ist Voraussetzung, daß die eingesetzten Calciumverbindungen wasserlöslich sein müssen. Dies kann zu Kompatibilitätsproblemen in dem häufig recht heterogen aufgebauten System einer Zahnpasta führen, so daß es umfangreicher galenischer Entwicklungsarbeit bedarf, eine entsprechend zusammengesetzte, stabile Zahnpasta herzustellen. Darüber hinaus kann der Zusatz von solchen Substanzen auch Geschmacksprobleme hervorrufen.

Es wurde nun gefunden, daß man auf einfache Weise ein ständiges Reservoir von Calciumionen, das beim Kontakt mit dem Zahnschmelz zur Verfügung steht, in einer Zahnpasta bereitstellen kann, wenn man ein Poliermittel einsetzt, das zu mindestens 20 Gew.-% aus Calciumcarbonat besteht und den pH-Wert der Zahnpasta durch Zusatz einer Säure auf etwa 7,5 bis etwa 8,5 absenkt.

Gegenstand der vorliegenden Erfindung sind also Zahnpasten, deren Putzkörper zu mindestens 20 Gew.-% aus Calciumcarbonat besteht und die zusätzlich eine Säure in einer solchen Konzentration enthalten, daß der pH-Wert dieser Paste zwischen 7,5 und 8,5 liegt. Derartige Zahnpasten weisen überraschenderweise eine ausgezeichnete remineralisierende Wirksamkeit auf, die derjenigen, die durch Zusatz löslicher Calciumverbindungen erzielt wird, zumindest entspricht oder sie teilweise sogar übertrifft.

Calciumcarbonat ist lange Zeit das in Zahnpasten meistgebrauchte Poliermittel gewesen und wird, obwohl teilweise durch andere Putzkörper wie verschiedene Calciumphosphate, Siliciumdioxid verschiedener Modifikationen oder Aluminiumhydroxid teilweise substituiert, auch heute noch in großem Umfang eingesetzt. Zahnpasten, die Calciumcarbonat enthalten, weisen jedoch einen pH-Wert auf, der bei 9 und höher liegt. Dabei liegt das Calciumcarbonat in vollkommen wasserunlöslicher Form vor, so daß eine Remineralisierung mit solchen Zahnpasten nicht erfolgen kann.

Es war daher überraschend und nicht vorhersehbar, daß durch die Absenkung des pH-Wertes in einen genau definierten Bereich zwischen 7,5 und 8,5 durch Säurezusatz eine kontrollierte Freigabe von Calciumionen und damit eine remineralisierende Wirkung auf den Zahnschmelz erreicht werden kann, ohne die sonstigen Eigenschaften einer solchen Zahnpasta zu beeinträchtigen.

Es ist dabei wichtig, daß jeweils nur soviel Säure zugesetzt wird, um den pH-Wert auf mindestens 8,5, jedoch nicht weniger als etwa 7,5 abzusenken, da sonst entweder keine Freisetzung von Calciumionen erreicht oder das Calciumcarbonat, bei zu niederem pH-Wert, vollständig zu dem entsprechenden Calciumsalz und Kohlensäure umgesetzt wird.

Die Menge der zuzusetzenden Säure ist selbstverständlich von deren Acidität abhängig und kann vom Fachmann in Kenntnis der Lehre der Erfindung durch einfaches Ausprobieren für jede Zahnpastazusammensetzung empirisch ermittelt werden.

Für diesen Zweck ist grundsätzlich jede physiologisch verträgliche Säure geeignet.

Im Rahmen der vorliegenden Erfindung einsetzbare organische Säuren sind beispielsweise Weinsäure, Citronensäure, Milchsäure, Apfelsäure, Benzoesäure, Glycerophosphorsäure, Gluconsäure, Glucuronsäure, Zimtsäure, Propionsäure, Ameisensäure, Bernsteinsäure, Malein- und Fumarsäure, Sorbinsäure und Isobuttersäure, Salicylsäure oder Valeriansäure.

Besonders bevorzugt wird hierbei der Zusatz von Weinsäure.

Als besonders geeignete anorganische Säure hat sich Salzsäure erwiesen.

Anstelle der freien Säure können selbstverständlich auch deren saure Salze eingesetzt werden, soweit sie geeignet sind, Calciumionen in Lösung zu halten.

Es soll an dieser Stelle darauf hingewiesen werden, daß der Zusatz von Säure zu Zahnpasten, auch zu solchen, die Calciumcarbonat als Poliermittel enthalten, an sich bereits vorgeschlagen wurde. Dies erfolgte jedoch zu einem anderen Zweck, so daß die erfindungsgemäß gegebene Lehre zum technischen Handeln, nämlich durch den Zusatz von Säure zur Einstellung eines bestimmten pH-Wertes Calciumcarbonat enthaltender Zahnpasten einen ganz bestimmten Effekt zu erzielen, aus diesem Stand der Technik weder bekannt noch herleitbar ist.

Es ist möglich, andere Poliermittel in geeigneten Mengen zuzusetzen, beispielsweise die verschiedenen Calciumphosphate, wie Dicalciumorthophosphat, Calciumpyrophosphat oder Tricalciumphosphat, Aluminiumhydroxid, Alkalialuminiumsilikate wie synthetisches Zeolith A, oder die verschiedenen Siliciumdioxid-Modifikationen, wobei die Menge des weiteren Poliermittels durch das Erfordernis der pH-Wert-Einstellung durch Säure vorgegeben ist. Das bedeutet, daß bei nicht alkalischen Poliermitteln der Anteil des Calciumcarbonats in der Regel überwiegt, während bei alkalischen Poliermitteln wie Zeolith A das Calciumcarbonat auch in einer Menge von weniger als 50 % des Gesamtpoliermittels vorliegen kann.

Der Gesamtanteil des Poliermittels in den erfindungsgemäßen Zahnpasten beträgt üblicherweise etwa 20 bis etwa 60, vorzugsweise etwa 25 bis etwa 55 Gew.-% der Gesamtzusammensetzung.

Die erfindungsgemäßen Zahnpasten enthalten, wie üblich, Feuchthaltemittel, vorzugsweise in einer Menge zwischen etwa 10 und 40, vorzugsweise etwa 15 bis 30 Gew.-%.

Geeignete Feuchthaltemittel sind beispielsweise Glycerin, Propylenglykol, 1,4-Butandiol, Sorbit, Mannit, Xylit oder Polyethylenglykole niederen Molekulargewichts.

Ebenso enthalten Zahnpasten üblicherweise Verdickungsmittel, vorzugsweise in einer Menge zwischen etwa 0,5 und 2,5 Gew.-% der Gesamtzusammensetzung.

Bevorzugte Verdickungsmittel sind Carboxymethylcellulose und deren Alkalisalze, insbesondere Natriumcarboxymethylcellulose, Hydroxyalkylcellulosen wie Hydroxymethylcellulose und Hydroxyethylcellulose, Methylcellulose, Pflanzengummen wie Tragant, Gummi arabicum, Carayagummi, Guargummi, Xanthangummi und Irish Moos, synthetische Polyelektrolyte wie die Al kalisalze der Polyacrylsäure sowie anorganische Verdickungsmittel, beispielsweise kolloidales Magnesiumaluminiumsilikat oder disperses Siliciumoxid.

Auch oberflächenaktive Stoffe können anwesend sein, vorzugsweise in einer Menge zwischen etwa 0,5 und 2,5 Gew.-% der Gesamtzusammensetzung.

Solche synthetischen oberflächenaktiven Stoffe sind beispielsweise Alkylsulfate, Alkylethersulfate, Olefinsulfonate, Natriumlauroylsarcosinat oder ampholytische, nicht-ionische oder kationaktive Verbindungen oder auch Seifen wie beispielsweise solche von Laurinsäure, Myristinsäure, Palmitinsäure, Stearinsäure oder Gemischen derselben, beispielsweise Cocosölfettsäuren oder Talgfettsäuren.

In den erfindungsgemäßen Zahnpasten können selbstverständlich auch weitere Wirkstoffe Verwendung finden. Solche sind insbesondere die bekannten kariesprophylaktischen Fluoride, vorzugsweise in einer solchen Menge, daß die Konzentration an reinem Fluor im Mittel etwa 0,05 bis etwa 1 Gew.-%, vorzugsweise 0,1 bis 0,5 Gew.-% des Mittels beträgt.

Geeignete Fluorverbindungen sind insbesondere die verschiedenen Salze der Monofluorphosphorsäure wie Natrium-, Kalium-, Lithium-, Calcium- und Aluminiummono- und difluorphosphat sowie, vorzugsweise im Gemisch mit Monofluorphosphaten, verschiedene, Fluor in ionisch gebundener Form enthaltende Fluoride, insbesondere Alkalifluoride wie Natrium-, Lithium-, Kalium- und Ammoniumfluorid, Zinnfluorid, Manganfluorid, Kupferfluorid, Zirkoniumfluorid und Aluminiumfluorid sowie Gemische oder Anlagerungsprodukte dieser Fluoride untereinander und mit anderen Fluorverbindungen, beispielsweise Alkalimanganfluoride.

Weitere, in den erfindungsgemäßen Zahnpasten einsetzbare Stoffe sind Zahnbelag entfernende Substanzen, beispielsweise Zink- und Kupfersalze, Mittel zur Verhinderung von Zahnsteinbildung wie Hydroxyethan-1,1-diphosphonsäure oder Alkylendiaminotetramethylenphosphonsäuren und deren wasserlösliche Salze, Allantoin, Azulen, etc..

Eine Übersicht über die Zusammensetzung und Herstellung von Zahnpasten, auf die hier ausdrücklich Bezug genommen werden soll, findet sich in der Monographie von M.S. Balsam und E. Sagarin, "Cosmetics - Science and Technology", 2nd Ed., Vol. 2, S. 423-531 (1972).

Im folgenden werden einige Beispiele für erfindungsgemäß zusammengesetzte Zahnpasten gegeben:

## Beispiel 1

| | |
|---|---|
| Calciumcarbonat | 11,00 (Gew.-%) |
| Zeolith A | 12,00 |
| (Natriumaluminiumsilikat) | |
| Glycerin | 9,80 |
| Sorbit, 70%-ig | 10,00 |
| Natriumcarboxymethylcellulose | 1,80 |
| Benzoesäure | 0,30 |
| Saccharin-Natrium | 0,15 |
| Natriummonofluorphosphat | 1,20 |
| Natriumlaurylsulfat | 1,80 |
| p-Hydroxybenzoesäureester | 0,25 |
| Kolloidales Silciumdioxid | 2,80 |
| Aromamischung | 1,00 |
| Citronensäuremonohydrat | 0,58 |
| Wasser | 48,22 |

pH-Wert der Paste: 8,1

4

## Beispiel 2

| | |
|---|---|
| Calciumcarbonat | 11,00 (Gew.-%) |
| Zeolith A | 12,00 |
| (Martisorb[R]; Natriumaluminium- | |
| silikat) | |
| Glycerin | 9,80 |
| Sorbit, 70%-ig | 10,00 |
| Natriumcarboxymethylcellulose | 1,80 |
| Benzoesäure | 0,30 |
| Saccharin-Natrium | 0,12 |
| Natriummonofluorphosphat | 1,20 |
| Natriumlaurylsulfat | 1,60 |
| p-Hydroxybenzoesäureester | 0,25 |
| Kolloidales Siliciumdioxid | 2,60 |
| Aromagemisch | 1,00 |
| Weinsäure | 0,65 |
| Wasser | 48,28 |

pH-Wert der Paste: 7,9

## Beispiel 3

| | |
|---|---|
| Calciumcarbonat | 11,00 (Gew.-%) |
| Zeolith A | 12,00 |
| (Sasil[R]; Natriumaluminiumsilikat) | |
| Glycerin | 9,80 |
| Sorbit, 7%ig | 10,00 |
| Natriumcarboxymethylcellulose | 1,70 |
| Benzoesäure | 0,30 |
| Saccharin-Natrium | 0,10 |
| Natriummonofluorphosphat | 0,80 |
| Natriumlaurylsulfat | 1,80 |
| p-Hydroxybenzoesäureester | 0,25 |
| Kolloidales Siliciumdioxid | 3,00 |
| Aromagemisch | 1,10 |
| Salzsäure, 10%-ig | 6,80 |
| Wasser | 41,95 |

pH-Wert der Paste: 7,7

### Beispiel 4

| | |
|---|---|
| Calciumcarbonat | 35,00 (Gew.-%) |
| Kolloidales Siliciumdioxid | 2,40 |
| Sorbit, 70%-ig | 10,00 |
| Natriumcarboxymethylcellulose | 1,00 |
| Hydroxyethylcellulose | 0,20 |
| Natriummonofluorphosphat | 0,80 |
| p-Hydroxybenzoesäureester | 0,25 |
| Saccharin-Natrium | 0,10 |
| Natriumlaurylsulfat | 1,60 |
| Allantoin | 0,20 |
| Aromagemisch | 1,00 |
| Gluconsäure, 80%-ig | 1,20 |
| Wasser | 46,25 |

pH-Wert der Paste: 7,8

### Beispiel 5

| | |
|---|---|
| Calciumcarbonat | 35,00 (Gew.-%) |
| Kolloidales Siliciumdioxid | 2,60 |
| Sorbit, 70%-ig | 10,00 |
| Natriumcarboxymethylcellulose | 1,30 |
| Natriummonofluorphosphat | 1,20 |
| p-Hydroxybenzoesäureester | 0,30 |
| Saccharin-Natrium | 0,08 |
| Natriumlaurylsulfat | 1,75 |
| Allantoin | 0,20 |
| Aromagemisch | 1,10 |
| Weinsäure | 0,45 |
| Wasser | 46,02 |

pH-Wert der Paste: 7,6

EP 0 190 762 B1

## Beispiel 6

| | |
|---|---|
| Calciumcarbonat | 35,00 (Gew.-%) |
| Kolloidales Siliciumdioxid | 2,60 |
| Sorbit, 70%-ig | 10,00 |
| Natriumcarboxymethylcellulose | 1,20 |
| Hydroxyethylcellulose | 0,15 |
| Natriummonofluorphosphat | 0,80 |
| p-Hydroxybenzoesäureester | 0,30 |
| Saccharin-Natrium | 0,10 |
| Natriumlaurylsulfat | 1,60 |
| Allantoin | 0,20 |
| Aromagemisch | 1,00 |
| Glycerinphosphorsäure, 50%-ig | 0,65 |
| Wasser | ad 100,00 |

pH-Wert der Paste: 7,8

**Patentansprüche**

1. Zahnpasta auf wäßriger Basis, die einen alkalischen pH-Wert aufweist, enthaltend ein Poliermittel, das zu mindestens 20 Gew.-% aus Calciumcarbonat besteht, dadurch gekennzeichnet, daß der pH-Wert durch Säurezusatz auf etwa 7,5 bis 8,5 eingestellt ist und die Zahnpasta eine zur Erzielung einer remineralisierenden Wirkung ausreichende Menge an verfügbaren Calciumionen enthält.

2. Zahnpasta nach Anspruch 1, dadurch gekennzeichnet, daß die Einstellung des pH-Wertes durch Weinsäure erfolgt ist.

3. Zahnpasta nach Anspruch 1 oder 2, dadurch gekennzeichnet, daß sie Calciumcarbonat als alleiniges oder überwiegendes Poliermittel enthält.

**Claims**

1. Toothpaste on an aqueous basis which has an alkaline pH and contains a polishing agent which consists to the extent of at least 20% by weight of calcium carbonate, characterised in that the pH is adjusted to about 7.5 to 8.5 by addition of acid and the toothpaste contains an amount of available calcium ions which is sufficient to achieve a remineralising action.

2. Toothpaste according to Claim 1, characterised in that the pH is adjusted by tartaric acid.

3. Toothpaste according to Claim 1 or 2, characterised in that it contains calcium carbonate as the sole or predominant polishing agent.

**Revendications**

1. Pâte dentifrice à base aqueuse qui présente un pH alcalin, contenant un abrasif qui est constitué à

7

raison d'au moins 20% en poids par du carbonate de calcium, caractérisée en ce que le pH est ajusté par addition d'acide entre environ 7,5 et 8,5 et que la pâte dentifrice contient une quantité suffisante d'ions calcium disponibles pour l'obtention d'un effet reminéralisant.

2.  Pâte dentifrice selon la revendication 1, caractérisée en ce que l'ajustement du pH s'opère avec de l'acide tartrique.

3.  Pâte dentifrice selon la revendication 1 ou 2, caractérisée en ce qu'elle contient du carbonate de calcium en tant qu'abrasif unique ou prédominant.